# NEUE EUROPÄISCHE PATENTSCHRIFT

(11) **EP 1 646 844 B2**
(45) Veröffentlichungstag und Bekanntmachung des Hinweises auf die Entscheidung über den Einspruch: **08.08.2012**
(45) Hinweis auf die Patenterteilung: 19.08.2009
(21) Anmeldenummer: 04738037.3
(22) Anmeldetag: 25.06.2004
(51) Int. Cl.: G01D 5/16, A61M 5/315

(54) **INJEKTIONSGERÄT MIT EINEM MAGNETORESISTIVEN SENSOR**
INJECTION APPARATUS HAVING A MAGNETORESISTIVE SENSOR
APPAREIL D'INJECTION AYANT UN CAPTEUR MAGNÉTORÉSISTIF

(30) Priorität: 09.07.2003 DE 10330986
(43) Veröffentlichungstag der Anmeldung: 19.04.2006
(73) Patentinhaber: TecPharma Licensing AG, 3401 Burgdorf (CH)
(72) Erfinder: STEFFEN, Beat, CH-3792 SAANEN (CH)
(74) Vertreter: Gassenhuber, Andreas
(86) Internationale Anmeldenummer: PCT/CH2004/000396
(87) Internationale Veröffentlichungsnummer: WO 2005/005929

(56) Entgegenhaltungen:
- EP-A- 0 425 690
- EP-A1- 1 095 668
- WO-A-02/064196
- WO-A1-02//064196
- WO-A1-03//011374
- DE-A- 19 907 716
- FR-A- 2 600 258
- US-A- 4 369 780
- US-A- 4 652 260
- US-A- 4 786 870
- US-A- 4 880 011
- US-A- 5 649 810
- US-A- 5 869 962
- US-A- 5 998 989
- US-A- 6 019 745
- US-B1- 6 456 063
- PATENT ABSTRACTS OF JAPAN Bd. 012, Nr. 200 (E-619), 9. Juni 1988 (1988-06-09) -& JP 63 001345 A (SEIKO EPSON CORP), 6. Januar 1988 (1988-01-06)
- H-R TRÄNKLER ET AL: 'Sensortechnik', 1998, SPRINGER-VERLAG, BERLIN, ISBN 3-540-58640-7
- 'The Embo Journal', Bd. 40, teil 2 22 Januar 1991, IRL PRESS LIMITED, DE, ISSN 0013-5658 Artikel PETERSEN A.: 'Drehzahlmessung mit magnetoresistiven Sensormodulen, hybrider Sensorbaustein fertig abgeglichen für den Einbau.', Seiten 78 - 81
- 'The Embo Journal', Bd. 41, teil 24 24 November 1992, IRL PRESS LIMITED, DE, ISSN 0013-5658 Artikel GRAEGER V. ET AL: 'Magnetoresistiver Drehzahlsensor,"zuverlässig und preiswert"', Seiten 48 - 52

## Beschreibung

Die vorliegende Erfindung betrifft eine Vorrichtung zur Verabreichung eines fluiden Produkts, insbesondere ein Injektionsgerät, mit einer Messeinrichtung zur berührungslosen Messung einer Stellung zwischen relativ zueinander beweglichen Elementen innerhalb der Verabreichungsvorrichtung und ein Verfahren für eine solche Messung. Insbesondere betrifft die Erfindung die Messung der Einstellung einer Verabreichungs- oder Dosiereinrichtung der Verabreichungsvorrichtung.

Vorrichtungen, wie sie der Erfindung zu Grunde liegen, finden in weiten Bereichen der Medizin zur Verabreichung eines medizinischen oder pharmazeutischen fluiden Produkts Anwendung. Beispielsweise werden Injektionsgeräte, wie etwa ein Injektionspen, zur Abgabe von Insulin, Hormonpräparaten und dergleichen verwendet. Ein Injektionsgerät weist verschiedene mechanische Einrichtungen, wie etwa eine Verabreichungs- oder Dosiereinrichtung auf, um z. B. eine bestimmte Produktdosis exakt aus dem Gerät abgeben zu können. Um den Verabreichungsvorgang und seine Genauigkeit kontrollieren zu können, ist es üblich innerhalb des Geräts Sensoren oder Taster anzuordnen, welche die Bewegung verschiedener Elemente der mechanischen Einrichtungen erfassen. Daraus wird z. B. mittels eines Mikroprozessors die Einstellung der mechanischen Einrichtungen ermittelt und kann z. B. durch eine mechanische oder elektronische Anzeige an dem Injektionsgerät angegeben werden.

Da eine mechanische Abtastung anfällig für Verschmutzung, Feuchtigkeit und Abnutzung ist und große Toleranzen zwischen den einzelnen Elementen aufweist, wodurch die Genauigkeit der Messung der Einstellung eines Injeldionsgeräts eingeschränkt wird, sind berührungslose Verfahren zur Bestimmung der Einstellung eines solchen Geräts entwickelt worden. Hierfür werden mehrere Sensoren oder Messvorrichtungen an verschiedenen Stellen des Geräts angeordnet, die zur Messung der Einstellung geeignet sind, ohne dass die Elemente dabei mit den Messvorrichtungen oder Sensoren in Kontakt kommen.

Aus der EP 1095668 A1 ist z. B. ein elektronischer Verabreichungspen für medizinische Zwecke bekannt, der zur Messung der Einstellung einer Verabreichungseinrichtung des Pens z.B. die lineare Position einer Schraubenstange des Verabreichungsmechanismus oder die Drehposition eines Einstellknopfes einer Dosiereinrichtung misst. Hierfür wird z.B. ein optischer Codeumwandler mit einer Codescheibe verwendet, die an die Drehbewegung des Einstellknopfes gekoppelt ist. Die Drehbewegung der Codescheibe wird von einem optischen Empfänger gemessen. Die Anzahl der Drehungen der Codescheibe wird von einem Mikroprozessor in eine der Einstellung entsprechende Dosismenge umgesetzt. Ein weiterer Sensor ist zwischen den Windungen der Schraubenstange der Verabreichungseinrichtung vorgesehen und registriert die Bewegung in Längsrichtung entlang der Längsachse des Pens. Aus der Verschiebung der Schraubenstange wird die verabreichte Menge eines Produkts bestimmt. Die beiden Sensoren arbeiten unabhängig voneinander und bestimmen jeweils nur eine Bewegungsrichtung einer mechanischen Einrichtung des Pens.

Durch derartige Messeinrichtungen zur berührungslosen Messung kann zwar die Genauigkeit der Messung einer Einstellung gegenüber einer mechanischen Abtastung erhöht werden, jedoch ist die Anordnung der Einzelteile einer solchen Messeinrichtung innerhalb des Geräts oftmals komplex, so dass die Herstellung des Geräts aufwendig und kostspielig ist. Die Verschaltungen und Messmethoden dieser Messeinrichtungen sind zudem anfällig für Feuchtigkeit, Vibrationen und andere derartige Einflüsse. Die Unterbringung der Einzelteile der Messeinrichtung, wie der Sensoren und der Gegenstücke für die Sensoren, erfordern häufig bauliche Veränderungen in dem Injektionsgerät, wodurch dieses unnötig groß wird oder gar die übrigen mechanischen Einrichtungen des Geräts beeinträchtigt werden.

Aus der WO 02/064196 A1 ist ferner ein Injektionsgerät bekannt, das durch eine geschlossene Schalteinheit mit integrierten Sensoren gesteuert wird, die ausgewählte Parameter des Geräts überwachen. Die abgeschlossene Schalteinheit ist feststehend innerhalb des Injektionsgeräts angeordnet. Als Sensoren werden wenigstens zwei Paare von integrierten Hall-Elementen verwendet. Die Hall-Elemente arbeiten mit einem magnetisierten Ring zusammen, der abwechselnd Nord- und Südpole aufweist Der Ring ist innerhalb einer Dosiereinrichtung angeordnet und wird in Übereinstimmung mit einer Drehbewegung zur Einstellung einer Produktdosis um die Längsachse des Injektionsgeräts bewegt. Um das Volumen einer Dosiseinstellung zu messen, ist es erforderlich, die Drehbewegung des magnetischen Rings relativ zu der abgeschlossenen Schalteinheit zu bestimmen. Hierfür werden die Hall-Elemente in einer definierten Zuordnung zueinander und zu dem magnetischen Ring auf einem Kreisbogen angeordnet, der dem magnetischen Ring gegenüberliegt. Beim Start der Bewegung wird ein Startwinkel definiert und auf der Grundlage der Messung des Magnetfeldes während der Bewegung des Magnetrings gegenüber den Hall-Elementen ein Endwinkel nach dem Abschluss der Bewegung bestimmt. Die Anfangs- und Endwinkel und das gemessene magnetische Feld werden mit einer gespeicherten Tabelle verglichen und aus dem Vergleich eine eingestellte Produktdosis bestimmt.

Die US 6 456 063 B1 aus dem technischen Gebiet des Automobilbaus bezieht sich auf Gegenstände, wie eine Kurbelwelle oder Nockenwellen. Exakte Positionen sind für das Programmieren von Motorsteuergeräten irrelevant, denn durch das Auflegen des Steuerriemens oder der Steuerkette ist die Absolutposition des Steuertriebs der Maschine schon im Montagezustand permanent festgelegt. Die US 6 456 063 B1 lehrt nachteiligerweise, dass die Messsignale von magnetoresistiven Sensoren sehr unzuverlässig sind.

Die EP 0 425 690 A1 beschreibt eine Methode zur Detektion von Betriebszuständen einer Mikropipette, wobei Positionsbeziehungen mittels geeigneter Sensoren detektiert werden. Als brauchbar werden optisch gekoppelte, mechanische und magnetische Sensoren und deren Kombination genannt. Wie die Realisierung tatsächlich auszuführen ist, wird in der Druckschrift nicht näher erörtert.

Die US 4 369 780 nutzt ein magnetoresistives Element zusammen mit einem Magneten lediglich als Sicherheitskomponente für den Fall des Ausfalles eines Mikroschalters. Nachteiligerweise lehrt die US 4 369 780 die Nutzung des magnetoresistiven Elementes nur zur Detektion der Linearverschiebung des Magneten.

Es ist eine Aufgabe der vorliegenden Erfindung, ein Injektionsgerät zur Verabreichung eines fluiden Produkts mit einer Messeinrichtung zur berührungslosen Messung einer Stellung einer Dosier- oder Verabreichungseinrichtung des Injektionsgeräts und ein Verfahren für eine solche berührungslose Messung bereitzustellen, wobei das Injektionsgerät einen einfachen Aufbau der Messeinrichtung mit wenigen zur Messung notwendigen Einzelteilen aufweist, insbesondere sollen bauliche Gegebenheiten des Injektionsgeräts zur Messung ausgenutzt werden können. Ferner soll eine hohe Genauigkeit und Zuverlässigkeit bei der Messung gewährleistet, eine Störanfälligkeit verhindert und die Herstellungskosten reduziert werden.

Diese Aufgabe wird durch ein Injektionsgerät zur Verabreichung eines fluiden Produkts nach Anspruch 1 und durch ein Verfahren nach Anspruch 12 erfüllt. Vorteilhafte Ausgestaltungen gehen aus den Unteransprüchen hervor.

Ein Injektionsgerät, wie es der vorliegenden Erfindung zugrunde liegt, weist verschiedene mechanische Einrichtungen, wie etwa eine Verabreichungs- oder Dosiereinrichtung auf, die aus mehreren Elementen aufgebaut sind, die sich bei der Handhabung des Geräts relativ zueinander innerhalb des Geräts bewegen. Beispielsweise wird zur Verabreichung eines Produkts aus dem Gerät ein Schubelement, wie z. B. eine Zahnstange, entlang der Längsachse des Geräts relativ zu einem Produktbehälter, einem Gerätegehäuse oder weiteren Führungseleinenten der Verabreichungseinrichtung bewegt. Eine Dosiereinrichtung zur Einstellung eines Dosisvolumens für ein zu verabreichenden fluides Produkt umfasst z. B. ein Drehelement, das relativ zum Gehäuse oder einer Gewindestange gedreht wird. Erfindungsgemäß weist das Injektionsgerät eine Messeinrichtung auf, welche durch die Bestimmung der Relativbewegung dieser Elemente zueinander die Einstellung einer mechanischen Einrichtung und damit des Injektionsgeräts misst.

Hierfür weist die Messeinrichtung wenigstens einen magnetoresistiven Sensor und wenigstens eine magnetische Vorrichtung auf. Der magnetoresistive Sensor ist an einem ersten Element des Injektionsgeräts befestigt und liegt einem relativ zum ersten Element beweglichen zweiten Element gegenüber. Das erste Element ist vorzugsweise das Gehäuse des Injektionsgeräts oder ein zu dem Gehäuse feststehendes Element. Das zweite Element ist bevorzugt ein Schub- oder Drehelement wie oben beschrieben, das gegenüber dem Gehäuse oder dem gehäusefesten Element bewegt wird.

Im Vergleich zu anderen Magnetfeldsensoren, wie induktiven Sensoren und Hall-Effektsensoren, weist der Einsatz von magnetoresistiven Sensoren in Injektionsgeräten mehrere Vorteile auf. Das Ausgangsmesssignal der magnetoresistiven Sensoren ist nicht von der Geschwindigkeit der Bewegung der Elemente zueinander abhängig und liefert auch bei sehr niedrigen Bewegungsgeschwindigkeiten ein zuverlässiges Messsignal, da eine Frequenz bis nahezu 0 Hz detektierbar ist. Im Vergleich zu Hall-Effektsensoren haben magnetoresistive Sensoren ein wesentlich stärkeres Ausgangssignal mit einer Signalamplitude von ungefähr 20 mV/kA/m. Ihre Empfindlichkeit ist um 10 bis 100 mal stärker. Hall-Effektsensoren haben typischerweise nur eine Signalamplitude von 0,4 mV/kA/m. Dadurch wird ein größerer Zwischenraum zwischen dem Messsensor und dem gegenüber diesem bewegten Element ermöglicht, so dass die Anordnungsmöglichkeiten für den Sensor und eine mit diesem zusammenwirkende magnetische Vorrichtung innerhalb eines Injektionsgeräts erweitert werden. Ferner können kostengünstigere magnetische Vorrichtungen, wie etwa einfache Stabmagneten oder Magnetstreifen, eingesetzt werden. Aufgrund ihrer hohen mechanischen Festigkeit sind magnetoresistive Sensoren auch leicht zu verarbeiten, universell einsetzbar und wenig störanfällig.

Magnetoresistive Sensoren nutzen den magnetoresistiven Effekt aus, wonach ein stromführendes magnetisches Material in Anwesenheit eines veränderlichen, externen Magnetfeldes seinen Widerstand entsprechend der Magnetfeldänderung ändert. Es wird grundsätzlich die Änderung des Magnetfeldes am Sensor durch die Bewegung der sich in dem Injektionsgerät gegenüberliegenden Elemente, d. h. durch ein Zusammenwirken der magnetischen Vorrichtung und des Sensors, erzeugt.

Erfindungsgemäß wird eine erste Art einer magnetischen Vorrichtung durch einen Permanentmagneten an dem ersten Element gemeinsam mit wenigstens einem zweiten Element gebildet, das magnetisierbar ist und ein vorbestimmtes Oberflächenprofil aufweist. Der Permanentmagnet ist z. B. ein einfacher zweipoliger Magnet. Vorzugsweise ist der Permanentmagnet am Sensor zwischen dem ersten und dem zweiten Element und besonders bevorzugt zwischen dem Sensor und dem zweiten Element angeordnet. Es ist vorteilhaft, als magnetisierbares Material im Sinne der Erfindung ein paramagnetisches Material, d. h. ein Material mit einer magnetischen Suszeptibilität größer als 1, zu verwenden. Besonders bevorzugt wird bei der Erfindung ein ferromagnetisches Material verwendet, wie z. B. Eisen, Kobalt, Nickel oder Gradolinium, da ein ferromagnetisches Material eine wesentlich größere magnetische Suszeptibilität als 1 besitzt. Durch die Anwesenheit des Permanentmagneten des ersten Elements werden die magnetischen Dipolmomente des ferromagnetischen Materials des zweiten Elements in Richtung des Magnetfeldes des Permanentmagneten ausgerichtet, d. h. das Material wird magnetisiert.

Das auf den am ersten Element angeordneten Sensor einwirkende effektive Magnetfeld setzt sich aus dem Magnetfeld des Permanentmagneten und dem Magnetfeld des ferromagnetischen zweiten Elements zusammen. Die Magnetisierung des ferromagnetischen zweiten Elements, bzw. dessen Einfluss auf das effektive Magnetfeld, hängt von dem Abstand der Oberfläche des zweiten Elements zu dem Permanentmagneten ab. Um eine Magnetfeldänderung und damit eine Widerstandsänderung an dem Sensor zu erzeugen, ist erfindungsgemäß das zweite Element mit einem vorbestimmten Oberflächenprofil ausgebildet, das sich bevorzugt aus zwei unterschiedlichen Höhenniveaus zusammensetzt, die sich periodisch abwechseln. Das vorbestimmte Oberflächenprofil kann z. B. durch Stufen oder Zacken auf der Oberfläche des zweiten Elements gebildet werden. Das vorbestimmte Oberflächenprofil des zweiten Elements ist dem Sensor und dem Magneten des ersten Elements gegenüberliegend angeordnet. Bei einer Bewegung des ersten Elements und des zweiten Elements gegeneinander wechselt daher der Abstand zwischen dem Sensor, bzw. dem Permanentmagnet und der Oberfläche des ferromagnetischen zweiten Elements periodisch, so dass sich das auf den Sensor einwirkende effektive Magnetfeld ebenfalls periodisch ändert. Diese Magnetfeldänderung erzeugt wiederum in dem Sensor eine messbare Widerstandsänderung, die als Messsignal ausgegeben werden kann. Die Abstände zwischen den unterschiedlichen Höhenniveaus des Oberflächenprofils können entsprechend einer gewünschten Widerstandsänderung gewählt werden. Dabei kann z. B. eine Periode der Widerstandsänderung als eine zurückgelegte Weglängeneinheit definiert werden. Durch die Aufsummierung der vom Sensor registrierten vollständigen oder begonnenen Weglängeneinheiten kann die gesamte bei der Bewegung zurückgelegte Wegstrecke bestimmt werden. Es ist deshalb möglich festzustellen, ob eine Dosis vollständig oder nur teilweise ausgeschüttet wurde, d.h. nicht der gesamte Verabreichungsweg zurückgelegt wurde.

Gemäß der vorliegenden Erfindung ist es besonders vorteilhaft, als Elemente mit einem vorbestimmten Oberflächenprofil der beschriebenen Form Elemente zu verwenden, die ohnehin zur Handhabung des Injektionsgeräts notwendig sind. Es ist daher nicht erforderlich, weitere Vorrichtungen wie die Magnetvorrichtung in dem Gerät unterzubringen oder die bestehenden Elemente zur Messung zu verändern. Es kann z. B. eine Zahnstange einer Verabreichungseinrichtung oder ein Zahnrad einer Dosiereinrichtung verwendet werden. Der Sensor kann dann an einem geeigneten der Zahnstange, bzw. dem Zahnrad, gegenüberliegenden Element angeordnet werden.

Eine zweite Art einer magnetischen Vorrichtung wird durch zumindest einen Permanentmagneten aus mehreren abwechselnd angeordneten Magnetpolbereichen gebildet, der an einem zweiten Element angeordnet ist. Daraus ergibt sich ein vektorielles Magnetfeld in Sinusform. Der Abstand und die Größe der Magnetpolbereiche kann dabei variiert werden. So ist es möglich die Stärke und den Verlauf des Magnetfeldes und damit die Ausgangssignalstärke des Sensors zu beeinflussen. Vorzugsweise wird ein derartiger Permanentmagnet als Magnetstreifen oder -ring ausgebildet. Der Magnetstreifen oder -ring wird derart an dem zweiten Element angebracht, dass er dem Sensor am ersten Element gegenüberliegt. Dabei wird der Abstand zwischen dem Sensor und dem Magnetstreifen oder -ring derart gewählt, dass das erzeugte Magnetfeld eine messbare Widerstandsänderung am Sensor erzeugt. Durch die Verwendung eines magnetoresistiven Sensors kann dieser Abstand jedoch in einem weiten Bereich variiert werden und bis zu einigen Milimetern betragen. Das zweite Element kann bei dieser Art der magnetischen Vorrichtung aus einem beliebigen Material bestehen, welches das Magnetfeld des Permanentmagneten nicht wesentlich beeinträchtigt.

Werden die beiden Elemente des Injektionsgeräts relativ zueinander bewegt, wie z. B. bei einer Dosiseinstellung oder bei einer Produktverabreichung, registriert der Sensor die sinusförmigen Schwankungen des Magnetfeldes als Widerstandsänderung. Dabei entspricht der Abstand zwischen einem Maximum und einem Minimum des Magnetfeldes z. B. einer zurückgelegten Weglängeneinheit. Durch die Aufsummierung der gemessenen Weglängeneinheiten kann wiederum die zurückgelegte Wegstrecke bestimmt werden.

Bei einem Verfahren gemäß der Erfindung zur berührungslosen Messung einer Stellung zwischen relativ zueinander bewegten Elementen einer oben beschriebenen Verabreichungsvorrichtung wird also an dem magnetoresistiven Sensor eine messbare Widerstandsänderung bei der Bewegung des ersten Elements gegenüber dem zweiten Element erzeugt, indem zum einen ein Magnetfeld eines am ersten Element angeordneten Permanentmagneten dadurch verändert wird, dass eine Oberfläche wenigstens eines magnetisierbaren zweiten Elements bei der Bewegung der Elemente gegeneinander ihren Abstand zu dem Permanentmagneten ändert. Zum anderen kann die Widerstandsänderung durch zumindest einen Permanentmagnet aus mehreren in Bewegungsrichtung abwechselnd angeordneten unterschiedlichen Magnetpolbereichen an wenigstens einem zweiten Element erzeugt werden. Bei dem Verfahren sind beide Möglichkeiten zur Erzeugung der Widerstandsänderung am Sensor einzeln oder miteinander einsetzbar. Auf der Grundlage der Widerstandsänderung wird die bei der Bewegung zurückgelegte Wegstrecke wie bereits dargestellt erfasst. Zur Ermittlung der Stellung der beweglichen Elemente zueinander wird die zurückgelegte Wegstrecke zu einer Referenzstellung der Verabreichungsvorrichtung in Bezug gesetzt. Hierfür kann z. B. das Ausgangsmesssignal des Sensors an einen Mikroprozessor abgegeben werden, der einen Wert einer Referenzstellung z. B. aus einem Speicher abruft und die neue Stellung bestimmt. Als Referenzstellung dient z. B. eine Ausgangsstellung der Elemente vor Beginn ihrer Bewegung relativ zueinander. Natürlich ist es auch möglich, mehrere Messeinheiten aus einem magnetoresistiven Sensor und einer erfindungsgemäßen magnetischen Vorrichtung in einer Verabreichungsvtirrichtung vorzusehen, um verschiedenste Einstellungen der Vorrichtung zu bestimmen oder die einzelnen Messsignale miteinander zu verarbeiten. Durch eine erfindungsgemäße Bestimmung der Stellung der beweglichen Elemente einer mechanischen Einrichtung der Verabreichungsvorrichtung kann z. B. die Dosiseinstellung eines zu verabreichenden fluiden Produkts oder der bei der Verabreichung zurückgelegte Verabreichungsweg bestimmt werden. Demnach kann die tatsächlich verabreichte Dosis erfasst werden, die sich unter Umständen von einer ausgewählten voreingestellten Dosis unterscheiden kann. Es kann also festgestellt werden, ob eine Dosisverabreichung vollständig oder nur teilweise erfolgt ist.

Mit Hilfe der Erfindung ist es auch möglich die Einstellung einer Verabreichungsvorrichtung, wie einem Injektionsgerät mittels diskreten Einstellpositionen anzugeben. Die diskreten Einstellpositionen sind z. B. durch die Periode der Widerstandsänderung in dem magnetoresistiven Sensor gegeben oder durch die Stellungen, die durch eine zurückgelegte Wegstrecke bestimmt sind, die aus ganzzahligen Vielfachen einer Weglängeneinheit besteht. Die periodische Widerstandsänderung wird dabei wie oben beschrieben durch eine magnetische Vorrichtung gemäß der Erfindung und durch die Bewegung der Elemente gegeneinander erzeugt. Dabei ist es möglich Referenzmesspunkte vorzusehen, indem z. B. das Oberflächenprofil wenigstens ein sich von den periodisch abwechselnden Höhenniveaus unterscheidendes Referenzhöhenniveau aufweist oder an einem Magnetstreifen oder -ring zusätzlich zu den unterschiedlichen Magnetpolbereichen ein Referenzpolbereich vorgesehen ist, der sich von den Magnetpolbereichen z. B. in der Stärke seiner Magnetisierung unterscheidet. Durch einen solchen Referenzpunkt weist die im Übrigen periodisch verlaufende Widerstandsänderung an dem Sensor z. B. eine besonders ausgeprägte Amplitude auf, wodurch eine bestimmte Einstellung des Injektionsgeräts wie etwa ein Ausgangs- oder Endpunkt definiert werden kann.

In einer Ausfürungsform eines Injektionsgerätes in Form eines Injektionspens gemäß der Erfindung, ist ein Sensor an einem ersten festen Element sowohl einem Schubelement als auch einem Drehelement gegenüberliegend vorgesehen. Vorzugsweise ist der Sensor gegenüber einer Zahnstange einer Verabreichungseinrichtung und einem Zahnrad einer Dosiereinrichtung angeordnet, so dass mit nur einem magnetoresistiven Sensor sowohl die Dosiseinstellung als auch die Verabreichungseinstellung eines Injektionspens bestimmt werden kann. Bei dieser Ausführungsform sind besonderes wenige Bauteile für eine erfindungsgemäße Messeinrichtung zur Bestimmung der Einstellung des Injektionspens erforderlichen.

Dabei werden diskrete Drehstellungen der Dosiereinrichtung derart ausgenutzt, dass bei jeder diskreten Dosiereinstellung eine Zahnreihe entlang der Zahnstange dem Sensor gegenüberliegt. Nimmt die Dosiereinrichtung eine solche Drehstellung ein, kann bei der Verabreichung der eingestellten Dosis die von der Zahnstange zurückgelegte Weglänge gemessen werden. Besonders vorteilhaft ist es dabei, dass durch die erfindungsgemäße Messeinrichtung der tatsächlich von der Zahnstange zurückgelegte Weg und damit das tatsächlich ausgeschüttete Volumen eines Produkts gemessen werden kann. Wird bei einer eingestellten Dosis nicht die vollständige Dosisweglänge überwunden, kann es zu einer Unterdosierung führen, eine solche Unterdosierung kann durch die erfindungsgemäße Messung der Einstellung registriert und einem Anwender rückgemeldet werden.

Die Messung einer Dreh- und einer Längsbewegung der Elemente relativ zueinander ist mit einer magnetischen Vorrichtung nach der zweiten Art möglich. Sind an dem zweiten Element mehrere Permanentmagneten in Form von Magnetstreifen oder -ringen angeordnet, sind diese Magnetstreifen oder -ringe ebenfalls an diskreten Einstellpositionen einer Längs- oder Drehstellung vorgesehen, so dass ausgehend von dieser diskreten Position eine weitere Messung einer anderen Bewegung entlang dieser Magnetstreifen oder -ringe erfolgen kann.

Weiter ist es mit der vorliegenden Erfindung möglich, die Bewegungsrichtung innerhalb einer Bewegungsdimension eines bewegten Elements zu erfassen, d. h. ob das Element in dieser Dimension vorwärts oder rückwärts bewegt wird. Hierfür kann eine charakteristische Periodizität des Magnetfeldes eines Magnetstreifens oder -rings oder des vorbestimmten Oberflächenprofils, bzw. der Widerstandsänderung, herangezogen werden. Weist der Verlauf der Widerstandsänderung z. B. Maxima mit einer steilen und einer flachen Flanke auf, kann aus der Abfolge der steilen und flachen Flanken die Bewegungsrichtung bestimmt werden. Ferner ist es möglich, die Richtung mit Hilfe von Magnetfeldern zu ermitteln, bei welchen Maximas mit unterschiedlicher Höhe ausgebildet sind. Es ist auch möglich zur Messung der Bewegungsrichtung zwei verschiedene Sensoren vorzusehen, deren Messsignale miteinander verarbeitet werden, wobei deren Widerstandsänderungen entsprechend einer Bewegungsrichtung miteinander in Beziehung stehen. Ferner ist es durch den Einsatz von zwei Sensoren möglich, festzustellen, ob sich ein Element entlang oder um die Längsachse oder radial zur Längsachse des Injektionsgeräts bewegt.

Letzlich ist es mit der vorliegenden Erfindung möglich, die Stellung von Elementen zu bestimmen, die sich in radialer Richtung der Längsachse eines Injektionsgeräts bewegen. Es kann z. B. die Stellung einer Veniegelungseinrichtung eines Injektionsgeräts gemessen werden. Dadurch kann z. B. festgestellt werden, ob eine Zahnstange einer Verabreichungsvorrichtung vollstandig entsprechend einer eingestellten Dosis vorgeschoben wurde, bis sie eine Veniegelungsposition erreicht hat. Hierfür ist z. B. ein magnetoresistiver Sensor an einem zum Gehäuse feststehenden ersten Element angebracht und liegt einem zweiten Element derart gegenüber, dass das zweite Element radial zur Längsachse des Injektionsgeräts auf den Sensors zu und von ihm weg beweglich ist. Es ist nicht erforderlich, den Sensor an der Oberfläche des zweiten Elements entlang zu führen. Ein solches zweites Element kann z. B. ein Verriegelungselement sein, das in einer ersten radialen Stellung das Injektionsgerät entriegelt und in einer zweiten radialen Stellung verriegelt. Das Entriegelungselement ist vorzugsweise aus ferromagnetischem Material. Bei der radialen Bewegung des Vemegelungsetements gegenüber dem Sensor verändert sich der Abstand zwischen dem Sensor und der Oberfläche des Verriegelungselements. Dadurch wird wie oben beschrieben ein Magnetfeld eines am Sensor angeordneten Permanentmagneten verändert und eine Widerstandsänderung in dem Sensor erzeugt, woraus die Stellung des Entriegelungselements ermittelt werden kann.

Durch den erfindungsgemäßen Einsatz eines magnetoresistiven Sensors und einer erfindungsgemäßen magnetischen Vorrichtung eröffnet sich eine Vielzahl von Möglichkeiten für den Aufbau einer Messeinrichtung für eine Verabreichungsvorrichtung, ohne auf eine zuverlässige und exakte Messung zu verzichten. Insbesondere ist es möglich, die vorhandenen Bauelemente eines Geräts zur Messung zu nutzen und einen magnetoresistiven Sensor an einer zur Messung besonders geeigneten Stelle innerhalb der Verabreichungsvorrichtung anzuordnen. Die aufgeführte Möglichkeiten sollen den Umfang der Erfindung nicht einschränken.

Ein Ausführungsbeispiel einer erfindungsgemäßen Verabreichungsvorrichtung wird anhand der Zeichnung näher erläutert; in dieser stellen dar:
- Figur 1:: einen Längsschnitt durch einen hinteren Bereich eines erfindungsgemäßen Injektionsgeräts,
- Figuren 2a und 2b:: einen Querschnitt durch ein Drehelement und
- Figuren 3a und 3b:: einen Querschnitt durch eine Verriegelungseinrichtung eines erfindungsgemäßen Injektionsgeräts.

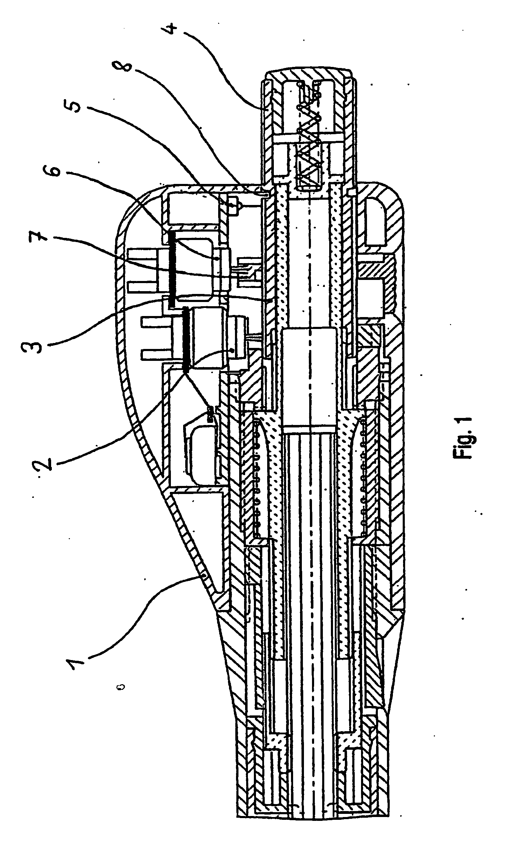

In Fig. 1 ist der hintere Bereich eines Injektionsgerätes mit den wesentlichen Teilen einer Dosier- und einer Verabreichungseinrichtung dargestellt. Die einzelnen Elemente sind innerhalb eines Gehäuses 1 untergebracht. Ein erster magnetoresistiver Sensor 2 ist innerhalb des Injektionsgeräts mit dem Gehäuse 1 fest verbunden, so dass das Gehäuse 1 ein erstes Element im Sinne der Erfindung darstellt. Der magnetoresistive Sensor 2 ist gegenüber einer Hülse 3 angeordnet, die gegenüber dem Gehäuse 1 um die Längsachse des Injektionsgerätes drehbar und entlang der Längsachse verschiebbar ist. Die Hülse 3 stellt erfindungsgemäß ein zweites Element dar, das gegenüber dem ersten Element beweglich ist. Die Hülse 3 dient dabei sowohl als Drehelement der Dosiereinrichtung zur Einstellung einer zu verabreichenden Produktdosis, als auch als Schubelement für den Vorschub eines Kolbens innerhalb eines Produktreservoirs (nicht gezeigt). Zur Verabreichung eines Produkts wird die Hülse zuerst entsprechend einer gewünschten Dosis an ihrem aus dem Injektionsgerät herausragenden Ende 4 gedreht und anschließend durch Eindrücken des Endes 4 in das Injektionsgerät vorgeschoben.

Ebenfalls gegenüber der Hülse 3 ist eine Lichtschranke 5 angeordnet. Die Lichtschranke 5 dient in Verbindung mit dem magnetoresistiven Sensor 2 zur Bestimmung der Bewegungsrichtung der Hülse 3, d. h. ob die Hülse in Umfangsrichtung oder in Längsrichtung gegenüber dem Gehäuse 1 bewegt wird. Als Lichtschranke 5 können herkömmliche Bauelement verwendet werden, die vorzugsweise energiesparend ausgebildet sind. Eine andere Möglichkeit eine Bewegungsrichtung festzustellen ist durch die Anordnung von Schaltern gegeben, wie etwa Reed-Kontakte, mechanische Schalter etc.

Ein zweiter magnetoresistiver Sensor 6 ist ebenfalls fest mit dem Gehäuse 1 verbunden. Der zweite Sensor 6 ist gegenüber einem beweglichen Element 7 einer Verriegelungseinrichtung des Injektionsgeräts angeordnet. Das bewegliche Element 7 der Verriegelungseinrichtung ist durch einen Resetring gegeben, der in entriegeltem Zustand einen Vorschub des Schubelements ermöglicht und in verriegeltem Zustand eine solche Bewegung verhindert. Hierfür ist der Resetring 7 in radialer Richtung zur Längsachse des Injektionsgeräts verschiebbar und greift zur Verriegelung in eine Nut 8 an der Hülse 3 ein. Der Resetring 7 bildet daher ebenfalls ein zweites Element im Sinne der vorliegenden Erfindung.

Als magnetoresistive Sensoren können z. B. Sensoren der Baureihe KMI 15/X von "Philips" verwendet werden. Natürlich ist es möglich, auch magnetoresistive Sensoren anderer Hersteller für die vorliegende Erfindung zu verwenden. Der erste magnetoresistive Sensor 2, der zweite magnetoresistive Sensor 6 und die Lichtschranke 5 sind an einen Mikroprozessor angeschlossen, der die Signale der Sensoren 2 und 6 und der Lichtschranke 5 sammelt und verarbeitet. Der Mikroprozessor ist ebenfalls innerhalb des Gehäuses 1 des Injektionsgeräts in der Nähe der Sensoren befestigt. Es ist nicht notwendig, dass die Sensoren und der Mikroprozessor als ein gemeinsames geschlossenes Bauelement ausgebildet sind. Dadurch können die Sensoren innerhalb des Injektionsgeräts an einer für die Messung besonders geeigneten Stelle angeordnet werden.

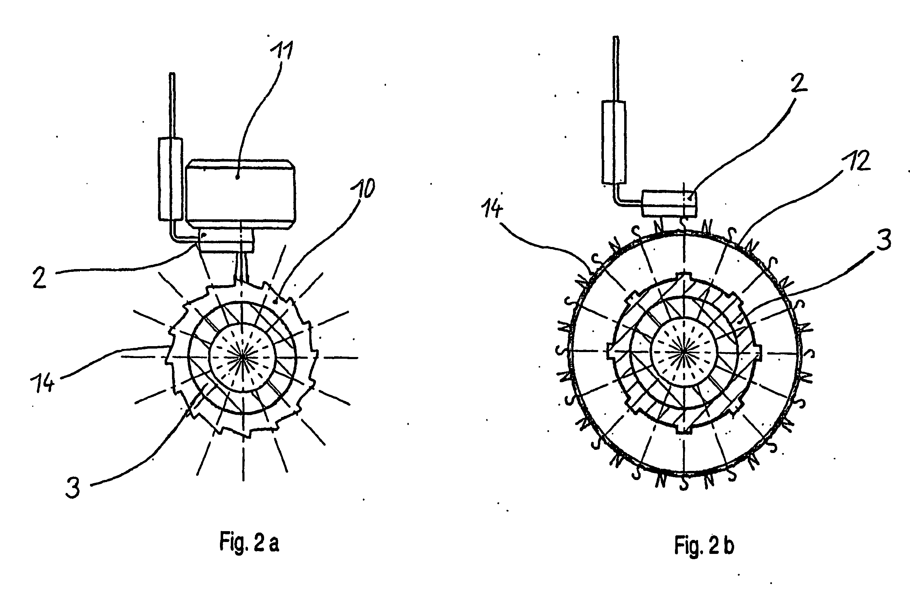

In Figur 2a ist ein Querschnitt durch eine Hülse 3 mit einem vorbestimmten Oberflächenprofil 14 gezeigt. Das Oberflächenprofil 14 wird durch ein Zahnrad 10 auf der Hülse 3 ausgebildet. Durch die Zähne des Oberflächenprofils werden diskrete Drehstellungen der Hülse 3 definiert, die einer bestimmten eingestellten Dosismenge entsprechen. Entsprechend bestimmter diskreten Drehstellungen der Hülse 3 sind anschließend an das Zahnrad entlang der Oberfläche der Hülse 3 in Längsrichtung des Injektionsgerätes Magnetstreifen mit mehreren abwechselnd angeordneten unterschiedlich gepolten Magnetpolbereichen angeordnet (in der Zeichnung nicht dargestellt).

Nimmt die Hülse 3 eine der bestimmten diskreten Drehpositionen ein und wird sie anschließend in Vorschubrichtung in das Injektionsgerät vorgeschoben, wird der zu dieser Drehposition gehörende Magnetstreifen entlang dem Sensor verschoben, so dass dieser nach der Drehposition auch die Wegstrecke der Verschiebung messen kann. Entsprechend dem Abstand der unterschiedlichen Magnetpolbereiche auf dem Magnetstreifen können auch in Längsrichtung diskrete Einstellpositionen bestimmt sein. Sobald die Hülse 3 in Vorschubrichtung bewegt wird, kann die Lichtschranke 5 diese Längsbewegung z. B. dadurch registrieren, dass die Nut 8 zu Beginn des Vorschubs an ihr vorbeigeschoben wird.

Oberhalb des Zahnrads 10 ist der erste magnetoresistive Sensor 2 angeordnet. Über dem magnetoresistiven Sensor 2 ist ein Permanentmagnet 11 angeordnet. Der Permanentmagnet 11 ist daher auch an dem ersten Element, dem Gehäuse 1, befestigt. Der Permanentmagnet 11 erzeugt von sich aus ein unveränderliches Magnetfeld, das in der Zeichnung durch Pfeile in Richtung des Zahnrades 10 dargestellt ist. Das Zahnrad 10 ist aus einem magnetisierbaren Material vorzugsweise aus einem ferromagnetischen Material hergestellt, wie etwa Eisen, Nickel, Kobalt oder Legierungen davon mit einer Suszeptibilitätskonstante µ > > 1. Das vorbestimmte Oberflächenprofil 14 des Zahnrades 10 besteht aus mehreren Zähnen, die in gleichem Abstand angeordnet sind und die auf einer, Seite eine radial verlaufende steile Flanke und auf der anderen Seite eine flach abfallende Flanke aufweisen. Dadurch ergibt sich ein Oberflächenprofil 14 mit einem ersten Höhenniveau zwischen den Zähnen und einem zweiten Höhenniveau an den Spitzen der Zähne. Bei einer Drehbewegung des Zahnrades 10 durch die Hülse 3 vorbei an dem Sensor variiert daher der Abstand zwischen der Oberfläche des Zahnrads 10 und dem Sensor 2 periodisch entsprechend dieser beiden Höhenniveaus. Das Magnetfeld des Magneten 11 wird bei verschiedenen Abständen zwischen dem Zahnrad 10 und dem Sensor 2 unterschiedlich abgelenkt und beeinflusst so das auf den Sensor wirkende effektive Magnetfeld, so dass eine Widerstandsänderung in dem Sensor 2 entsteht. Durch die asymmetrische Form der Zähne des Zahnrades 10 mit einer steilen und einer flachen Flanke ist es auch möglich, die Drehrichtung des Zahnrades 10 und damit der Hülse 3 zu bestimmen. Durch die Bestimmung der Drehrichtung, kann auch eine Dosisanpassung, z. B. durch ein Zurückbewegen der Dosiereinrichtung erkannt und korrekt durchgeführt werden.

In Fig. 2b ist eine alternative Ausgdirungsform der Hülse 3 gezeigt. Die dem Sensor 2 gegenüberliegende Umfangsfläche der Hülse 3 ist mit einem Permanentmagnetring 12 versehen, der mehrere abwechselnd angeordnete unterschiedliche Magnetpolbereiche aufweist, d. h. Nord- und Sildpolbereiche, die mit N und S bezeichnet sind. Bei dieser Ausführungsform des Injektionsgerätes ist kein Permanentmagnet 11 erforderlich. Ein solcher Magnetring 12 kann nach der Herstellung der Hülse auf diese aufgebracht werden. Die alternierenden Pole des Magnetrings erzeugen bei einer Drehbewegung der Hülse 3 ein sinusförmiges Magnetfeld an dem Sensor 2 und damit einer Widerstandsänderung an dem Sensor 2. Der Abstand zwischen den Magnetpolbereichen N und S kann entsprechend dem Auflösungsvermögen des Sensors gewählt werden. Bei herkömmlich erhältlichen magnetoresistiven Sensoren ist ein so kleiner Abstand möglich, dass bereits kleine Weglängen, bzw. ein kleines Bogenmaß, gemessen werden können. Es ist daher eine sehr präzise Messung der Einstellung des Injektionsgeräts möglich. Ebenso ist es möglich, die Abstände der Magnetpolbereiche N und S entsprechend diskreten Einstellpositionen zu wählen, die im Allgemeinen größer sind als der Abstand, der von dem Sensor noch messbar ist.

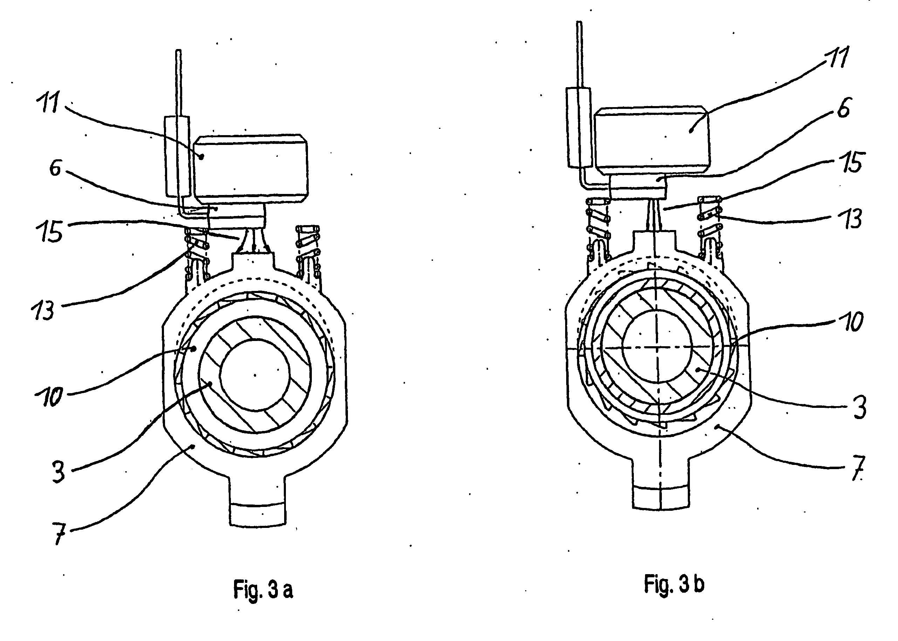

In Fig. 3a ist ein Querschnitt durch eine Verriegelungseinrichtung des erfindungsgemäßen Injektionsgeräts in einer entriegelten Position dargestellt. Die Oberfläche des Resetrings 7 ist gegenüber dem Sensor 6 in einem ersten Abstand angeordnet. Über dem magnetoresistiven Sensor 6 ist ein Permanentmagnet 11 befestigt, der ein Magnetfeld entsprechend der eingezeichneten Pfeile in Richtung des Resetrings 7 erzeugt. Am Ende einer Vorschubbewegung der Hülse 3 wird der Resetring 7 durch eine Federkraft von vorgespannten Federn 13 in die Nut 8 gedrückt. Dadurch wird der Abstand zwischen der Oberfläche des Resetrings 7 und dem Sensor 6 von dem ersten Abstand auf einen zweiten größeren Abstand geändert. Diese Abstandsänderung zwischen dem Resetring 7 und dem Sensor 6 ändert auch den Feldlinienverlauf des effektiven Magnetfeldes 15, das auf den Sensor 6 einwirkt, und damit den Widerstand innerhalb dem Sensor 6, wie in Fig. 3b durch die Vektoren des Magnetfeldes gezeigt ist. Dadurch kann die Verriegelungsstellung der Verriegelungseinrichtung des Injektionsgeräts gemessen werden. Ein besonderes Oberflächenprofil ist auf der Oberfläche des Resetrings 7 nicht notwendig, da sich eine Abstandsänderung zwischen dem ersten und dem zweiten Element allein durch die Bewegung der Elemente relativ zueinander ergibt.

Entsprechend den gezeigten Anordnungen von Sensoren und bewegten Elementen kann eine Vielzahl von Bewegungsabläufen innerhalb einer Verabreichungsvorrichtung gemessen und damit die Einstellung der Vorrichtung bestimmt werden. Die dargestellte Anordnung ist daher beispielhaft zu verstehen und soll den Umfang der Erfindung nicht einschränken.

### Bezugszeichen

- 1: Gehäuse
- 2: erster magnetoresistiver Sensor
- 3: Hülse
- 4: Hülsenende
- 5: Lichtschranke
- 6: zweiter magnetoresistiver Sensor
- 7: Resetring
- 8: Nut
- 9: -
- 10: Zahnrad
- 11: Permanentmagnet
- 12: Permanentmagnetring
- 13: Feder
- 14: Oberflächenprofil
- 15: Feldlinien
- N: positiver Magnetpolbereich
- S: negativer Magnetpolbereich

## Patentansprüche

1. Injektionsgerät zur Verabreichung eines fluiden Produkts, insbesondere eines medizinischen oder pharmazeutischen fluiden Produkts, mit einer Messeinrichtung zur berührungslosen Messung einer Stellung zwischen relativ zueinander beweglichen Elementen (1; 3) einer Dosier- oder Verabreichungseinrichtung des Injektionsgeräts, wobei die Messeinrichtung aufweist:
a) wenigstens einen magnetoresistiven Sensor (2; 6), der an einem ersten Element (1) befestigt und wenigstens einem relativ zum ersten Element (1) beweglichen als Drehelement ausgebildeten magnetisierbaren zweiten Element (3) gegenüberliegend angeordnet ist zum Messen einer Drehposition, und
b) eine magnetische Vorrichtung, die gebildet wird aus:
- einem Permanentmagneten (11) am ersten Element ( 1 ) und
- wenigstens dem magnetisierbaren zweiten Element (3) mit einem derart vorbestimmten Oberflächenprofil (14), dass bei der Bewegung der Elemente (1; 3) zueinander eine Oberfläche des zweiten Elements (3) ihren Abstand zu dem Permanentmagneten (11) ändert, wodurch aufgrund einer erzeugten Magnetfeldänderung in dem magnetoresistiven Sensor (2; 6) eine messbare Widerstandsänderung erzeugt wird;
wobei ein Oberflächenprofil (14) des magnetisierbaren zweiten Elements (3) wenigstens zwei sich periodisch abwechselnde Höhenniveaus umfasst, und so eingerichtet ist, dass der Verlauf der Widerstandsänderung im magnetoresistiven Sensor (2; 6) Maxima mit einer steilen und flachen Flanke aufweist, so dass aus der Abfolge der steilen und flachen Flanken eine Bewegungsrichtung bestimmt werden kann.
wobei das zweite Element (3) ein Drehelement ist, das um die Längsachse des Injektionsgeräts zur Verabreichung relativ zu dem ersten Element (1) drehbar ist.

2. Injektionsgerät zur Verabreichung eines fluiden Produkts nach dem vorhergehenden Anspruch, wobei der Permanentmagnet (11) am ersten Element (1) ein Stabmagnet ist.

3. Injektionsgerät zur Verabreichung eines fluiden Produkts nach einem der vorhergehenden Ansprüche, wobei der Permanentmagnet (11) am ersten Element (1) am magnetoresistiven Sensor (2; 6) angeordnet ist.

4. Injektionsgerät zur Verabreichung eines fluiden Produkts nach einem der vorhergehenden Ansprüche, wobei das magnetisierbare zweite Element (3) ferromagnetisch ist.

5. Injektionsgerät zur Verabreichung eines fluiden Produkts nach einem der vorhergehen Ansprüche, wobei das Oberflächenprofil (14) wenigstens ein sich von den periodisch abwechselnden Höhenniveaus unterscheidendes Referenzhöhenniveau aufweist.

6. Injektionsgerät zur Verabreichung eines fluiden Produkts nach einem der vorhergehenden Ansprüche, wobei ein Permanentmagnet (12) an dem zweiten Element (3) ein Magnetstreifen oder -ring ist.

7. Injektionsgerät zur Verabreichung eines fluiden Produkts nach einem der vorhergehenden Ansprüche, wobei ein erstes Element von einem Gehäuse (1) des Geräts gebildet wird oder bezüglich des Gehäuses (1) feststeht.

8. Injektionsgerät zur Verabreichung eines fluiden Produkts nach einem der vorhergehenden Ansprüche, wobei ein magnetoresistiver Sensor (2; 6) sowohl einem Schubelement als auch dem Drehelement gegenüberliegt.

9. Injektionsgerät zur Verabreichung eines fluiden Produkts nach einem der vorhergehenden Ansprüche, wobei diskrete Einstellpositionen entsprechend dem vorbestimmten Oberflächenprofil bestimmt sind.

10. Injektionsgerät zur Verabreichung eines fluiden Produkts nach einem der vorhergehenden Ansprüche, wobei entlang der Längsachse auf dem Umfang eines Schubelements mehrere Magnetstreifen an Umfangpositionen vorgesehen sind, die diskreten Dreheinstellungen des Drehelements entsprechen.

11. Injektionsgerät zur Verabreichung eines fluiden Produkts nach einem der vorhergehenden Ansprüche, wobei eines der relativ zueinander beweglichen Teile (1; 3) an einer Verriegelungseinrichtung der Dosier- oder Verabreichungseinrichtung des Injektionsgeräts vorgesehen ist.

12. Verfahren zur berührungslosen Messung einer Stellung zwischen einem ersten Element (1) und einem zweiten Element (3), die relativ zueinander bewegliche Elemente (1; 3) sind, eines Injektionsgeräts zur Verabreichung eines fluiden Produkts mit einer Messeinrichtung, die wenigstens einen magnetoresistiven Sensor (2; 6) aufweist, der an dem ersten Element (1) gegenüber wenigstens dem als Drehelement ausgebildeten zweiten Element (3) angeordnet ist zum Messen einer Drehposition, wobei
a) in dem magnetoresistiven Sensor (2; 6) eine messbare Widerstandsänderung bei der Bewegung der Elemente (1;3) relativ zueinander erzeugt wird,
indem ein Magnetfeld eines Permanentmagneten (11), der am ersten Element (1) angeordnet ist, **dadurch** verändert wird, dass eine Oberfläche wenigstens eines magnetisierbaren zweiten Elements (3) bei der Bewegung der Elemente (1;3) zueinander ihren Abstand zu dem Permanentmagneten (11) ändert, wobei ein Oberflächenprofil (14) des magnetisierbaren zweiten Elements (3) wenigstens zwei sich periodish abwechselnde Höhenniveaus umfasst, und so eingerichtet ist, dass der Verlauf der Widerstandsänderung im magnetoresistiven Sensor (2; 6) Maxima mit einer steilen und flachen Flanke aufweist, so dass aus der Abfolge der steilen und flachen Flanken eine Bewegungsrichtung bestimmt wird,
b) aufgrund der Widerstandsänderung eine bei der Bewegung zurückgelegte Wegstrecke erfasst wird und
c) zur Ermittlung der Stellung des ersten und wenigstens des zweiten Elements (1; 3) zueinander die zurückgelegte Wegstrecke zu einer Referenzstellung in Bezug gesetzt wird,
wobei das zweite Element (3) ein Drehelement ist, das um die Längsachse der Verabreichungsvorrichtung relativ zu dem ersten Element (1) drehbar ist.

13. Verfahren nach Anspruch 12, wobei eine Bewegungsrichtung eines bewegten Elements (3) entlang und/oder um die Längsachse und/oder radial zur Längsachse der Verabreichungsvorrichtung erfasst wird.

## Claims

1. An injection device for administering a fluid product, in particular a medical or pharmaceutical fluid product, comprising a measuring unit for measuring, in a non-contact process, a position between elements (1, 3) of a dosing or administering device of the injection device which can be moved relative to each other, said measuring unit comprising:
a) at least one magneto-resistive sensor (2; 6) which is fixed to a first element (1) and arranged opposite at least a second, magnetisable element (3) which can be moved relative to the first element (1) and is embodied as a rotational element, for measuring a rotational position; and
b) a magnetic device which is formed from:
- a permanent magnet (11) on the first element (1); and
- the at least second, magnetisable element (3) which has a predetermined surface profile (14) such that when the elements (1, 3) are moved relative to each other, a surface of the second element (3) changes its distance from the permanent magnet (11), whereby a measurable change in resistance is generated due to a generated change in the magnetic field in the magneto-resistive sensor (2; 6),
wherein a surface profile (14) of the magnetisable second element (3) includes at least two periodically alternating height levels and is configured such that the trajectory of the change in resistance in the magneto-resistive sensor (2; 6) exhibits maxima having one steep and one flat flank, such that a movement direction can be determined from the sequence of the steep and flat flanks,
wherein the second element (3) is a rotational element which can be rotated about the longitudinal axis of the injection device for administering, relative to the first element (1).

2. The injection device for administering a fluid product according to the preceding claim, wherein the permanent magnet (11) on the first element (1) is a rod magnet.

3. The injection device for administering a fluid product according to any one of the preceding claims, wherein the permanent magnet (11) on the first element (1) is arranged on the magneto-resistive sensor (2; 6).

4. The injection device for administering a fluid product according to any one of the preceding claims, wherein the magnetisable second element (3) is ferromagnetic.

5. The injection device for administering a fluid product according to any one of the preceding claims, wherein the surface profile (14) comprises at least one reference height level which differs from the periodically alternating height levels.

6. The injection device for administering a fluid product according to any one of the preceding claims, wherein a permanent magnet (12) on the second element (3) is a magnetic strip or ring.

7. The injection device for administering a fluid product according to any one of the preceding claims, wherein a first element is formed by a casing (1) of the device or is fixed relative to the casing (1).

8. The injection device for administering a fluid product according to any one of the preceding claims, wherein a magneto-resistive sensor (2; 6) is opposite both a thrust element and the rotational element.

9. The injection device for administering a fluid product according to any one of the preceding claims, wherein discrete setting positions are determined in accordance with the predetermined surface profile.

10. The injection device for administering a fluid product according to any one of the preceding claims, wherein a plurality of magnetic strips are provided along the longitudinal axis on the circumference of a thrust element at circumferential positions which correspond to discrete rotational settings of the rotational element.

11. The injection device for administering a fluid product according to any one of the preceding claims, wherein one of the parts (1; 3) which can be moved relative to each other is provided on a locking device of the dosing or administering device of the injection device.

12. A method for measuring, in a non-contact process, a position between a first element (1) and a second element (3) of an injection device for administering a fluid product, wherein the elements (1, 3) can be moved relative to each other and the injection device comprises a measuring unit which comprises at least one magneto-resistive sensor (2; 6) which is arranged on the first element (1) opposite at least the second element (3) which is embodied as a rotational element, for measuring a rotational position, wherein:
a) a measurable change in resistance in the magneto-resistive sensor (2; 6) is generated when the elements (1, 3) are moved relative to each other, by altering a magnetic field of a permanent magnet (11) which is arranged on the first element (1) by changing the distance between a surface of an at least second, magnetisable element (3) and the permanent magnet (11) when the elements (1, 3) are moved relative to each other, wherein a surface profile (14) of the magnetisable second element (3) includes at least two periodically alternating height levels and is configured such that the trajectory of the change in resistance in the magneto-resistive sensor (2; 6) exhibits maxima having one steep and one flat flank, such that a movement direction is determined from the sequence of the steep and flat flanks;
b) a path distance travelled during the movement is detected on the basis of the change in resistance; and
c) the path distance travelled is correlated with a reference position in order to ascertain the position of the first and at least second element (1, 3),
wherein the second element (3) is a rotational element which can be rotated about the longitudinal axis of the administering device, relative to the first element (1).

13. The method according to claim 12, wherein a direction of movement of a moved element (3) along and/or about the longitudinal axis and/or radially with respect to the longitudinal axis of the administering device is detected.

## Revendications

1. Dispositif d'injection pour administrer un produit liquide, notamment un produit liquide médicinal ou pharmaceutique, doté d'un dispositif de mesure pour mesurer sans contact une position entre des éléments (1; 3) mobiles l'un par rapport à l'autre d'un dispositif de dosage ou d'administration du dispositif d'injection, dans lequel le dispositif de mesure comporte:
a) au moins un capteur magnéto-résistant (2; 6), qui est fixé à un premier élément (1) et qui est disposé en face d'au moins un deuxième élément magnétisable (3) réalisé sous la forme d'un élément rotatif mobile par rapport au premier élément (1), pour mesurer une position de rotation, et
b) un dispositif magnétique, qui est formé par:
- un aimant permanent (11) sur le premier élément (1) et
- au moins le deuxième élément magnétisable (3) avec un profil de surface (14) prédéterminé de telle manière que, lors du déplacement des éléments (1; 3) l'un par rapport à l'autre une surface du deuxième élément (3) change de distance par rapport à l'aimant permanent (11), ce qui produit une variation de résistance mesurable à la suite d'une variation du champ magnétique produite dans le capteur magnéto-résistant (2; 6);
dans lequel un profil de surface (14) du deuxième élément magnétisable (3) comprend au moins deux niveaux de hauteur changeant périodiquement, et est réalisé de telle manière que l'évolution de la variation de résistance dans le capteur magnéto-résistant (2; 6) présente des maxima avec un flanc abrupt et plat, de telle manière qu'une direction de déplacement soit déterminée à partir de la succession de flancs abrupts et plats,
dans lequel le deuxième élément (3) est un élément rotatif, qui peut tourner par rapport au premier élément (1) autour de l'axe longitudinal du dispositif d'injection destiné à l'administration.

2. Dispositif d'injection pour l'administration d'un produit liquide selon la revendication précédente, dans lequel l'aimant permanent (11) sur le premier élément (1) est un aimant droit.

3. Dispositif d'injection pour l'administration d'un produit liquide selon l'une quelconque des revendications précédentes, dans lequel l'aimant permanent (11) sur le premier élément (1) est disposé sur le capteur magnéto-résistant (2; 6).

4. Dispositif d'injection pour l'administration d'un produit liquide selon l'une quelconque des revendications précédentes, dans lequel le deuxième élément magnétisable (3) est ferromagnétique.

5. Dispositif d'injection pour l'administration d'un produit liquide selon l'une quelconque des revendications précédentes, dans lequel le profil de surface (14) présente au moins un niveau de hauteur de référence se différenciant des niveaux de hauteur changeant périodiquement.

6. Dispositif d'injection pour l'administration d'un produit liquide selon l'une quelconque des revendications précédentes, dans lequel un aimant permanent (12) sur le deuxième élément (3) est une bande ou une bague magnétique.

7. Dispositif d'injection pour l'administration d'un produit liquide selon l'une quelconque des revendications précédentes, dans lequel un premier élément est constitué par un boîtier (1) du dispositif ou est fixé par rapport au boîtier (1).

8. Dispositif d'injection pour l'administration d'un produit liquide selon l'une quelconque des revendications précédentes, dans lequel un capteur magnéto-résistant (2; 6) fait face aussi bien à un élément de poussée qu'à l'élément rotatif.

9. Dispositif d'injection pour l'administration d'un produit liquide selon l'une quelconque des revendications précédentes, dans lequel des positions de réglage discrètes sont déterminées en fonction du profil de surface prédéterminé.

10. Dispositif d'injection pour l'administration d'un produit liquide selon l'une quelconque des revendications précédentes, dans lequel il est prévu le long de l'axe longitudinal, sur le périmètre d'un élément de poussée, plusieurs bandes magnétiques dans des positions périphériques qui correspondent à des réglages de rotation discrets de l'élément rotatif.

11. Dispositif d'injection pour l'administration d'un produit liquide selon l'une quelconque des revendications précédentes, dans lequel une des parties (1; 3) mobiles l'une par rapport à l'autre est prévue sur un dispositif de verrouillage du dispositif de dosage ou d'administration du dispositif d'injection.

12. Procédé pour mesurer sans contact une position entre un premier élément (1) et un deuxième élément (3), qui sont des éléments (1; 3) mobiles l'un par rapport à l'autre, d'un dispositif d'injection destiné à l'administration d'un produit liquide doté d'un dispositif de mesure, qui présente au moins un capteur magnéto-résistant (2; 6), qui est disposé sur le premier élément (1) en face d'au moins le deuxième élément (3) réalisé sous la forme d'un élément rotatif, pour la mesure d'une position de rotation, dans lequel
a) on produit dans le capteur magnéto-résistant (2; 6) une variation de résistance mesurable lors du déplacement des éléments (1; 3) l'un par rapport à l'autre,
du fait que l'on modifie un champ magnétique d'un aimant permanent (11), qui est disposé sur le premier élément (1), par le fait qu'une surface d'au moins un deuxième élément magnétisable (3) change de distance par rapport à l'aimant permanent (11) lors du déplacement des éléments (1; 3) l'un par rapport à l'autre,
dans lequel un profil de surface (14) du deuxième élément magnétisable (3) comprend au moins deux niveaux de hauteur changeant périodiquement, et est réalisé de telle manière que l'évolution de la variation de résistance dans le capteur magnéto-résistant (2; 6) présente des maxima avec un flanc abrupt et plat, de telle manière qu'une direction de déplacement soit déterminée à partir de la succession de flancs abrupts et plats,
b) on détecte un chemin parcouru lors du déplacement sur base de la variation de résistance, et
c) on compare le chemin parcouru à une position de référence pour déterminer la position du premier et au moins du deuxième élément (1; 3),
dans lequel le deuxième élément (3) est un élément rotatif, qui peut tourner par rapport au premier élément (1) autour de l'axe longitudinal du dispositif d'administration.

13. Procédé selon la revendication 12, dans lequel on détecte une direction de déplacement d'un élément déplacé (3) le long et/ou autour de l'axe longitudinal et/ou radialement à l'axe longitudinal du dispositif d'administration.
